# EUROPEAN PATENT APPLICATION

(11) **EP 4 623 966 A1**
(43) Date of publication of application: **01.10.2025**
(21) Application number: 24166773.2
(22) Date of filing: 27.03.2024
(51) Int. Cl.: A61M 11/00, A61M 15/00, A61M 16/00, A61M 16/20

(54) **INHALATION THERAPY DEVICE**

(71) Applicant: PARI Pharma GmbH, 82319 Starnberg (DE)
(72) Inventor: STOECKL, Carolin, 80331 München (DE); HOFFMANN, Tobias, 86938 Schondorf am Ammersee (DE); CARRASCO, Antonio, 81371 München (DE); LACHMAYR, Stefanie, 86415 Mering (DE)
(74) Representative: Hoffmann Eitle

(57) **Abstract**

The present disclosure refers to an inhalation therapy device. The inhalation therapy device comprises a housing body, a reservoir for holding a liquid, a membrane unit, and a valve. The membrane unit comprises an aerosol-generating membrane and an actuator. The aerosol-generating membrane is disposed in the housing body and comprises a plurality of apertures. The membrane is disposed so that, when liquid is held in the reservoir, the liquid is supplied to a first side of the aerosol-generating membrane by gravitational force. The actuator is coupled to the aerosol-generating membrane for vibrating the aerosol-generating membrane, whereby the liquid passes through the apertures, an aerosol is generated at a second side of the aerosol-generating membrane opposite to the first side of the aerosol-generating membrane, and a negative pressure builds up in the reservoir. The valve is configured to open when the amount of negative pressure in the reservoir reaches a threshold upon use of the inhalation therapy device, specifically upon vibration of the aerosol-generating membrane, and is configured to close when the amount of negative pressure drops below the threshold.

## Description

### Technical Field

The present disclosure relates to an inhalation therapy device for entraining and delivering a generated aerosol to a user or patient.

### Background Art

Inhalation therapy devices, also known as (inhalation) nebulizers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMAD (Mass Median Aerodynamic Diameter) of less than Spm or even less than 4µm and at least 1µm are required to allow deposition in the intended part of the respiratory system.

The aerosols for therapeutic purposes are generated and delivered to a desired location within the user's body, especially its respiratory tract, with the inhalation therapy device. To do so, a fluid, particularly a liquid, i.e., a medicament, a drug, a vaccine, etc., to be aerosolized or nebulized is supplied to a reservoir for holding the fluid or liquid in the inhalation therapy device.

For aerosolizing or nebulizing the liquid in the reservoir, the inhalation therapy device comprises an aerosol generator.

Such an aerosol generator may, for example, comprise a membrane unit, which comprises an aerosol-generating membrane having a plurality of apertures and an actuator that is directly or indirectly, via a support plate supporting the aerosol-generating membrane, coupled to the same for vibrating the aerosol-generating membrane.

The fluid or liquid may be in contact with a first side of the aerosol-generating membrane via gravitational force. The fluid or liquid, i.e., the medicament, passes through the apertures from the first side and an aerosol is generated at a second side of the aerosol-generating membrane opposite to the first side of the aerosol-generating membrane upon vibrating the same.

The aerosolized or nebulized fluid or liquid, i.e., the aerosol, inside the inhalation therapy device is then supplied to the user's respiratory system within the bounds of an inhalation therapy upon inhalation of the user through the inhalation therapy device. Meaning, upon the application of a suction force by the user's breathing, especially his or her inhalation through the inhalation therapy device, the aerosol is at least partially transported (by the suction force) from the interior of the inhalation therapy device to the user's respiratory tract.

An example for a conventional inhalation therapy device having such a configuration is, for example, derivable from DE 199 53 317 C1. The membrane unit of the disclosed inhalation therapy device comprises a cylindrical liquid reservoir container, which is delimited at one end face by an aerosol-generating membrane having the shape of a circular disc. A liquid disposed in the reservoir contacts the side of the aerosol-generating membrane facing the reservoir.

The aerosol-generating membrane disclosed in DE 199 53 317 C1 is connected (welded) to a substrate (support plate) and an oscillating generator, for example, a piezo crystal, which surrounds the aerosol-generating membrane in a circular manner and is connected (glued) to the substrate, such that the aerosol-generating membrane can be caused to oscillate by means of the oscillating generator and an electric drive circuit.

Thereby, the liquid applied to the aerosol-generating membrane on the first side of the same is conveyed through the apertures in the oscillating aerosol-generating membrane to the second side of the aerosol-generating membrane opposite to said first side to be emitted into a chamber as an aerosol. The disclosed inhalation therapy device further embodies a mouthpiece, or a mask connected to an outlet of the chamber and having an exhalation valve. Upon exhalation of a user, the exhaled flow will, thus, not or only to a limited extend enter the chamber and rather be expelled through the exhalation valve (or exhalation opening) into the environment of the device.

The disclosed device continuously drives the piezo crystal throughout the (single) treatment time (single administration interval), in which substantially the entire liquid in the reservoir is aerosolized by the aerosol-generating membrane. During exhalation of a user, the generated aerosol is temporarily stored in the chamber forming an aerosol cloud (bolus), which upon inhalation is inhaled and transported to the user's respiratory tract for therapy. Therefore, the treatment time can be kept relatively short.

One potential drawback is, however, that with an increasing output rate of the aerosol generator (aerosol-generating membrane, piezo crystal, drive circuit, etc.) the amount of aerosol deposited on the inner walls of the device, which is known as a so-called rainout, particularly in the chamber, increases. This leads to an increased loss of administrable aerosol/pharmaceutical.

Other conventionally known inhalation therapy devices drive the piezo crystal only during inhalation and stop driving the piezo crystal during exhalation. Those inhalation therapy devices are often referred to as breath-triggered devices or breath-actuated devices. It has been found that such inhalation therapy devices often have the issue of a long therapy duration (long administration interval), which can be annoying to the user and, thereby, provoke a lack of user adherence, e.g., an interruption or a premature end of the therapy before the intended dose of fluid or liquid in the reservoir has been nebulized and delivered to the user's respiratory tract for therapy.

Both of the conventional inhalation therapy devices described above are oftentimes operated in such a manner that the user merely inhales through the inhalation therapy device, i.e., an inhalation flow is generated through a flow path of the device, and the inhalation therapy device is removed from the user's mouth and/or nose upon exhalation. While both enables a resistance-free exhalation of the user, the overall treatment cannot be satisfactorily monitored, as no data, such as a flow parameter in a flow path of the device, preferably by detecting the pressure in the flow path, can be obtained during the exhalation. Additionally, and in case of a continuous operation, generated aerosol gets lost without any therapeutic effect for the user.

On top of that, removing the inhalation therapy device with every exhalation negatively affects the user's natural respiration, i.e., his or her steady breathing cycle.

Hence, conventional systems were confronted with a need for an effective inhalation therapy device that enables a short overall therapy time (single administration interval), while rainout and/or loss of generated aerosol without any therapeutic effect for the user can be reduced.

To achieve the same, various control mechanisms for the aerosol generation inside the inhalation therapy device have been proposed. While activating (during inhalation) and deactivating (during exhalation) the driving of the piezo crystal - in general - ensures that losses of the aerosolized fluid or liquid containing a pharmaceutical can be reduced, turning the aerosol generation on and off, i.e., performing the triggering at the right time is rather difficult.

On the one hand, the aerosol generation must start at the latest upon the start of the user's inhalation to omit an unnecessary prolongation of the treatment time. On the other hand, the production of aerosol must be stopped early enough that, upon exhalation of the user through the inhalation device, it can be avoided that produced aerosol is expelled from the inhalation therapy device and the user's upper respiratory tract without any therapeutic application.

To achieve the same, the so-called "pre-on/pre-off" inhalation therapy devices have been considered by the Applicant. Therein, the aerosol production is started in advance of the actual inhalation of the user, such that a certain amount of aerosol is generated before the actual inhalation, i.e., a pre-inhalation aerosol production similar to the aerosol bolus discussed above is produced, and the drive of the piezo crystal is stopped before the exhalation starts, i.e., a pre-exhalation aerosol production stop is performed.

To achieve the same, all non-continuously operating inhalation therapy devices require a precise monitoring of the user's breathing pattern at least in the inhalation direction to adequately control the drive of the piezo crystal and, thereby, the aerosol production.

Accordingly, most conventional non-continuously operating inhalation therapy devices comprise sensors. Such sensors observe the flows through the inhalation therapy device and are, therefore, necessary to precisely trigger the aerosol generation.

The previous solutions are, however, confronted with a deterioration of the aerosol generation, particular at the start and/or the end of a single administration interval, i.e., at the start and end of the overall therapy time, which results in rainout and/or loss of generated aerosol without any therapeutic effect for the user.

An example of a publication proposing a first solution in this field is derivable from WO 2012/069531 A2. The inhalation therapy device disclosed therein, according to its abstract, comprises a liquid reservoir defining in a sealed state a volume V_{R} configured to hold an initial volume of liquid V_{L} and an aerosol-generating membrane having openings. The liquid reservoir is connected to the aerosol-generating membrane to feed the liquid to one side of the aerosol-generating membrane. The aerosol-generating membrane is oscillatable to transport the liquid through the openings, whereby the liquid is emitted in the form of an aerosol on the other side of the aerosol-generating membrane. The volume V_{R} of the liquid reservoir before the aerosol-generating membrane is oscillated is configured to contain more than 5ml of gas at an initial volume of the liquid V_{L} of at least 5ml.

### Summary of the Disclosure

In view of the aforesaid, it is an object of the present disclosure to provide an inhalation therapy device that increases the overall quality of drug delivery in a consistent and reliable manner throughout the administration interval.

This object is solved by means of an inhalation therapy device according to independent claim 1. Distinct embodiments are derivable from the dependent claims.

According to a first aspect of the present disclosure, an inhalation therapy device comprises a housing body, a reservoir for holding a liquid, a membrane unit, and a valve.

The membrane unit comprises an actuator and an aerosol-generating membrane, which is disposed in the housing body and comprises a plurality of apertures. The aerosol-generating membrane is disposed so that, when liquid is held in the reservoir, the liquid is supplied to a first side of the aerosol-generating membrane by gravitational force.

The actuator is coupled to the aerosol-generating membrane for vibrating the same, whereby the liquid, which is held in the reservoir and is supplied to the first side of the aerosol-generating membrane by gravitational force, passes through the apertures, an aerosol is generated at a second side of the aerosol-generating membrane opposite to the first side of the aerosol-generating membrane, and a negative pressure builds up in the reservoir.

The valve is configured to open when the (amount of) negative pressure, which is also understood as underpressure, in the reservoir reaches a threshold upon/during use of the inhalation therapy device. Preferably, the valve is configured to open when the amount of negative pressure in the reservoir reaches the threshold upon/during vibration of the aerosol-generating membrane.

Further, the valve is configured to close when the (amount of) negative pressure drops below the threshold.

When the negative pressure ramps up during the administration interval, i.e., as it becomes more and more negative due to the decrease of the filling volume of the liquid in the reservoir caused by the aerosol generation via the membrane unit, the valve enables an exchange of air being locked in the reservoir with the environment, i.e., the outside of the reservoir once the threshold is hit. In this scenario, the valve opens and accordingly allows air to enter the reservoir. This results in a drop of the negative pressure, i.e., a reduction of the underpressure in the reservoir. Put differently, the pressure in the reservoir becomes less negative. Once the negative pressure inside the reservoir drops below the threshold, i.e., it has become less negative, the valve closes and a certain amount of negative pressure, which is below the threshold, can still be maintained - independent of the fluid filling volume in the reservoir.

The reservoir holds the liquid or fluid in a "sealed" state during therapy and the only opening for the liquid or fluid to exit the inhalation therapy device's reservoir during use of the same is via the apertures of the aerosol-generating membrane. At the same time, the valve ensures that the amount of negative pressure, which builds up during the therapy by more and more liquid being aerosolized by passing through the aerosol-generating membrane's apertures, is kept in an acceptable range, i.e., below a certain maximum pressure value, the above-noted threshold. Said mechanism and the accordingly caused presence and maintenance of the underpressure in a certain range enables that the liquid or fluid, i.e., the medicament, which is supplied to a first side of the aerosol-generating membrane by gravitational force, is aerosolized in its entirety and the delivered dose of the liquid or fluid, i.e., the medicament, can be maximized.

Preferably, the valve is a check valve.

This provides a simple configuration of the valve without the need for any electric, particularly sensory, components that trigger the opening and/or the closing of the valve. At the same time, the check valve effectively prevents any negative environmental influences from entering the reservoir in an undesired manner.

More preferably, the check valve is an umbrella valve that can adopt different opening pressures via the Shore hardness of its material. It therefore takes on the spring function of the valve itself. The term opening pressures is to be understood as the threshold value of the negative pressure, as in the outlines above. This means that the umbrella valve opens when the negative pressure becomes more negative and closes when the negative pressure decreases, i.e., becomes more positive (less negative).

Yet, the configuration of the check valve is not limited to such an umbrella valve. It may equally be possible to provide different configurations of predetermined pressure-dependent one-way valves. For example, a fixed valve with a spring also works, just as an electronically or mechanically (actively) controlled valve.

Preferably, the threshold of the amount of negative pressure is 100mbar or 200mbar of negative pressure.

It is to be understood that these values may slightly deviate, e.g., due to production variations, the material of the valve and its behaviour upon the triggering of the valve, or environmental conditions. That is, the described thresholds are to be understood as containing deviations of ± 25mbar or even ±50mbar, such that the threshold may be understood as a negative pressure range around the respective negative pressure values listed above.

Depending on the fluid or liquid to be used for the inhalation therapy, it may furthermore also be possible to use valve configurations having a threshold of up to -300mbar or even up to -400mbar of underpressure as the threshold for triggering the opening and closing of the valve.

As the present disclosure uses negative pressures values, i.e., underpressure values, as the trigger for opening and closing the valve, the threshold of the negative pressure in the reservoir that triggers the opening and also the closing of the valve is to be understood as being approximately -100mbar or -200mbar.

Having such a negative pressure trigger value enables that the delivered dose can be kept as high as possible and the largest amount of liquid, i.e., medicament, which is held in the reservoir, is aerosolized for therapy by the membrane unit.

Particularly, said negative pressure ensures that a "sucking" effect from the first side of the aerosol-generating membrane being oriented towards the interior of the reservoir to the second side of the aerosol-generating membrane inside the housing body is prevented. As such, difficulties in starting the nebulization, such as a "bleeding effect", wherein the fluid passes the apertures of the aerosol-generating membrane without being aerosolized, or the foam of liquid being held in the reservoir, can be omitted and the total output rate (TOR) can, thus, advantageously be increased.

With more and more liquid from the reservoir being aerosolized and, hence, being supplied from the first side of the aerosol-generating membrane to the second side of the aerosol-generating membrane, the negative pressure inside the reservoir builds up, whereas the threshold ensures that the amount of underpressure in the reservoir, i.e., on the first side of the membrane, does not exceed the respective thresholds given above that may vary depending on the to be aerosolized liquid or fluid.

At the same time, these trigger values for the closing and opening of the valve respectively ensure that a certain level of negative pressure is consistently maintained inside the reservoir during therapy, and it can be prevented that liquid passes the apertures of the aerosol-generating membrane without being used effectively as an aerosol in the aerosol therapy. If said negative pressure would not be given, liquid droplets or the like would be present at the second side of the aerosol-generating membrane inside the housing body without any therapeutic effect.

Preferably, the inhalation therapy device further comprises a lid for sealing an opening of the reservoir.

Said lid enables that the reservoir for holding the liquid can be easily accessed, and liquid can be refilled after opening the reservoir via the lid.

While such a (refill-)opening gets sealed by the lid, such that accidental spillage of the liquid can be prevented, gas can still enter the reservoir when the inhalation therapy device is, e.g., used without any liquid in the reservoir, because gas is capable to pass the apertures of the aerosol-generating membrane.

More preferably, the valve is arranged in the lid.

Accordingly, the reservoir itself can be shaped in a particularly simple and cost-effective form as a part of the inhalation therapy device, while the lid comprising the valve contains the major structural elements for creating and maintaining the negative pressure throughout the therapy interval.

Such an arrangement of the valve enables an integral configuration of the valve. However, the present disclosure is not limited to such a valve position. For example, the valve may also be arranged distant from the lid. It may, for example, be positioned in a side wall of the (fluid) reservoir or may be in fluid connection with the fluid reservoir via a piping structure. Yet, it must be kept in mind and taken care that the valve arrangement does not encounter liquid that gets filled into the (fluid) reservoir for being aerosolized, i.e., to be used for the inhalation therapy. That is, it its preferred to arrange the valve above the maximum liquid filling level of the reservoir when the inhalation therapy device is held in a normal position during its use for therapy in order to avoid any damages or pollution of the valve.

Preferably, the inhalation therapy device, more preferably the lid further comprises a negative pressure generating device for generating an initial negative pressure in the reservoir before the inhalation therapy device is operated, preferably before the aerosol-generating membrane of the inhalation therapy device is vibrated.

This enables that a negative pressure is existent in the (filled) reservoir already at the start of the aerosolization by the vibration of the aerosol-generating membrane. Given that said negative pressure is decisive for optimizing the amount of liquid usable for therapy due to the optimised conditions in the reservoir that are established by the negative pressure, the aerosol generation is, hence, further improved.

Furthermore, it can be prevented that an air volume, which is encapsuled between the liquid being held in the reservoir and the lid, provokes a positive pressure in the reservoir upon closing the lid, which could negatively affect the therapy success due to the occurrence of the above-discussed "bleeding" through the apertures of the aerosol-generating membrane.

It is, in this connection, to be understood that the initial negative pressure generation in the reservoir is independent of the actuation of the valve. Should, for whatever reason, a negative pressure amount be built up in the reservoir that exceeds the threshold, i.e., which is more negative than the predetermined negative pressure threshold of the valve, the valve will open to adjust the amount of negative pressure in the reservoir to a value that is less negative than the threshold. This is independent of the question, whether an initial pressure has been generated by the negative pressure generating device before the start of the therapy or not.

It is, in this regard, preferred that the lid comprises a lid body and that the negative pressure generating device comprises an annular sealing and a moving member.

The annular sealing is sandwiched at its outer circumferential portion between the lid body and a circumferential rim of the reservoir defining the opening upon placing of the lid on the reservoir.

The moving member is attached to an inner circumferential portion of the sealing. The moving member is configured to move the inner circumferential portion of the sealing away from the aerosol-generating membrane upon fastening of the lid body to the reservoir to generate the initial negative pressure.

This structural configuration enables to generate an initial negative pressure in the reservoir before the inhalation therapy starts, i.e., before the aerosol-generating membrane is vibrated to generate aerosol, without the need of any electric or mechanical-actuated elements. To the contrary, said configuration's shape enables to establish the negative pressure already before the aerosol generation starts in an easy, cost-effective, and reliable manner.

Thus, the therapy success can be improved, as the delivered dose can be kept as high as possible and the largest liquid amount possible can be used for therapy.

The valve may be arranged in the moving member.

This establishes a continuous negative pressure in the closed reservoir before and during the inhalation therapy with the above-described inhalation therapy device.

The interplay of the negative pressure generating device and the underpressure-sensitive valve being arranged in the lid enables that the two advantageous functions of creating a negative pressure before the start of therapy and maintaining the negative pressure in a preferred range, which does not have to be the same as the initial negative pressure, throughout the entire therapy can be achieved purely by the configuration of the lid.

As such, it may also be possible to update conventional membrane nebulizers with such a lid to achieve the above-described effects.

Further, said lid configuration allows to achieve an initial negative pressure and maintain the negative pressure, which does not have to be the same as the initial negative pressure, without any operational steps that need to be executed by an electrically operated controller or the like.

In addition, the arrangement of the valve in the lid, more preferably in the moving member, ensures that a contact of the liquid filled into the reservoir with the lid can be prevented, even when the inhalation therapy device is held with a 30° tilting angle compared to an ideal position when the inhalation therapy device is held by the user during therapy in a (standing or) sitting position of the user and an upright position of the user's head.

### Brief Description of the Drawings

Hereinafter, non-limiting examples of the disclosure are explained with reference to the drawings, in which:
- Figure 1: shows an embodiment of the inhalation therapy device according to the present disclosure in an isometric view.
- Figure 2: shows a schematic cross-sectional view along the flow path of the inhalation therapy device of Figure 1.
- Figure 3: shows an exploded view of the nebulizer housing of the inhalation therapy device of Figure 1.
- Figures 4a and 4b: show the operation states of the negative pressure generating device.
- Figure 5: provides a diagram, which exemplarily illustrates the negative pressure curve inside the reservoir in the inhalation therapy device over a single administration interval.

### Detailed Description of Preferred Embodiments

A currently preferred embodiment of the present disclosure regarding an inhalation therapy device 1 will now be described with reference to the accompanying drawings. Such inhalation therapy devices 1 are oftentimes also called "aerosol delivery devices" or "nebulizers", for example, for a therapeutic use via a user's respiratory system.

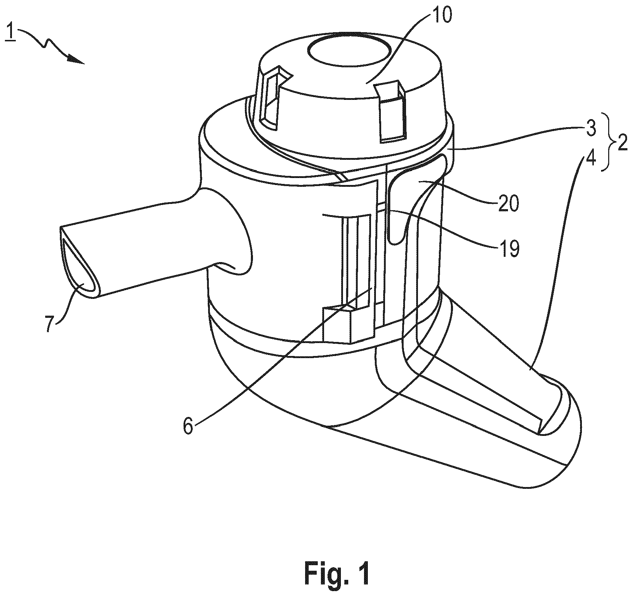

Fig. 1 shows an exemplary isometric view of the inhalation therapy device 1 according to a currently preferred embodiment of the present disclosure.

The overall shape of the inhalation therapy device 1 is defined by its housing body 2, which is split into two main parts. The housing body 2 comprises a nebulizer housing 3, which may equally be construed as an aerosolization portion, and a controller housing 4, which may also be called controller portion or holding portion.

Yet, the present disclosure is not limited to such a split configuration: It may also be possible to form the nebulizer housing 3 and the controller housing 4, which constitute the housing body 2 of the present embodiment, as an integral element. Such a configuration will, however, not be discussed in more detail here.

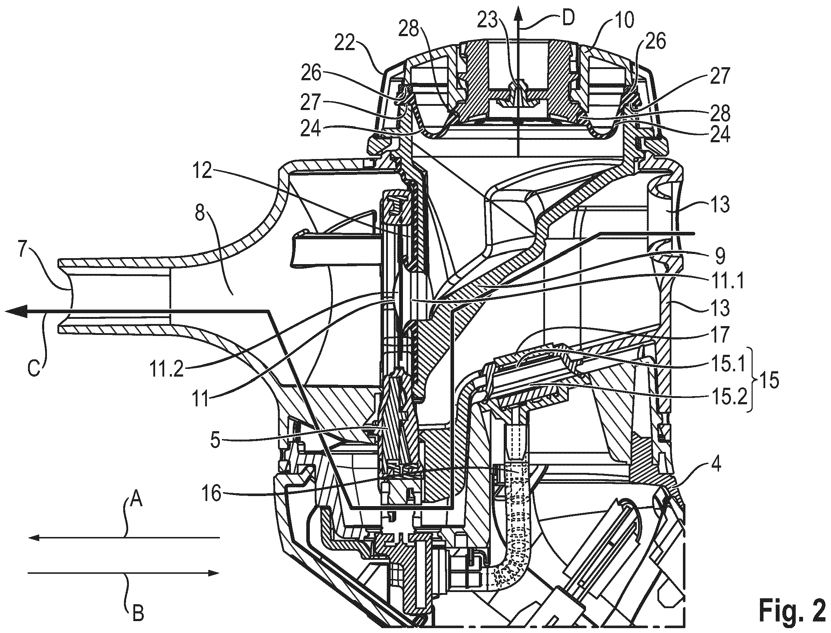

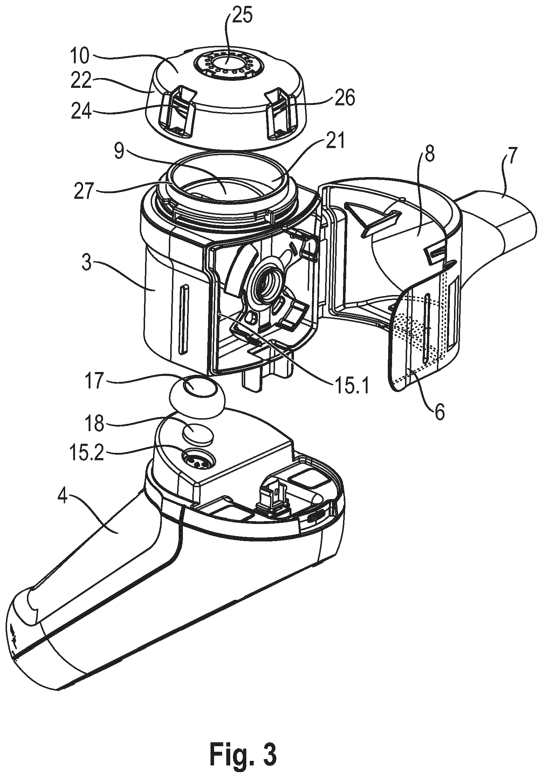

The nebulizer housing 3 accommodates a membrane unit 5, which is also known as an aerosol generator. The membrane unit 5 is configured to generate an aerosol from a liquid or fluid, i.e., a medicament. This will be described in more detail with reference to Figs. 2 and 3 below.

The controller housing 4 generally aims to enable that the inhalation therapy device 1 can be held by a user during (aerosol) therapy and contains the most relevant, preferably all, electronic features and sensory elements necessary to operate the inhalation therapy device 1 and to monitor the therapy process.

Accordingly, the controller housing 4 can be construed as a long-lasting element, which has lower requirements regarding cleaning and/or disinfection compared to the nebulizer housing 3, which can be configured as a throwaway product or a product, which is to be used for a certain amount of time, such as a certain number of therapies, weeks, or months.

Hitherto, the position of the nebulizer housing 3 relative to the controller housing 4 in a use state of the inhalation therapy device 1 is at least temporarily fixed via non-illustrated interlocking fasteners, and it is thereby also possible to reversibly attach and detach the nebulizer housing 3 from the controller housing 4 for the ease of cleanability of both parts of the housing body 2 and replacement of the nebulizer housing 3 via said interlocking fasteners, which may equally comprise magnetically interacting counterelements.

To enable a decent cleanability and/or disinfection of the nebulizer housing 3 and an easy access of the membrane unit 5 arranged inside the nebulizer housing 3, it is apparent from Figure 3 that a latch 6 is provided at the nebulizer housing 3. The latch 6 enables access to the interior of the nebulizer housing 3, especially to the membrane unit 5 arranged therein. Hitherto, Fig. 1 shows the inhalation therapy device 1 when the latch 6 holds the nebulizer housing 3 in a closed state and Fig. 3 shows the same in an open state, such that the (in Fig. 3 non-illustrated) membrane unit 5 can be accessed. Yet, providing the latch 6 is not compulsory. It is also possible to provide a nebulizer housing 3 that cannot be opened by a user, operator, hospital staff, or the patient itself. This is, for example, achievable by forming a nebulizer housing 3, in which the membrane unit 5 is arranged in a fixed position and cannot be accessed/replaced.

In the technical field given, such membrane units 5 are oftentimes known as membrane units or head units. Similarly, the nebulizer housing 3 is known as a "nebset", which reflects an abbreviation of nebulization set.

The preferred embodiment is an inhalation therapy device 1 through which a user inhales and exhales during the therapy. That is, the inhalation therapy device 1 is brought into contact with the user's mouth, preferably mouth and nose, for example, via a mask, such that the regular inhalation and exhalation of the user is performed through the inhalation therapy device 1. That is, the inhalation therapy device 1, specifically its first opening 7 (which will be described in more detail below) shall not be removed from the user's mouth (and nose) during the aerosol therapy. It, therefore, goes without saying that such an inhalation therapy device may equally be understood as an aerosol therapy device, which is applied during the inhalation and the exhalation of the user.

How the aerosol is generated inside the inhalation therapy device 1 and how the actual aerosol therapy is to be performed will now be described in more detail.

Fig. 2 illustrates the orientation of the inhalation therapy device 1 when it is held by a patient in an ideal position during therapy, i.e., in the sitting position of the patient and an upright position of the patient's head. For orientation purposes, solely the uppermost section of the controller housing 4, when the inhalation therapy device 1 is held in said ideal position, is illustrated. At the same time, Fig. 2 shows a cross-sectional view through the inhalation therapy device 1 along a flow path 8. It is, in this connection, illustrated that the nebulizer housing 3 contains the flow path 8, which passes through said nebulizer housing 3 and that the membrane unit 5 is disposed in said flow path 8.

In the illustrated position, a reservoir 9 for holding a liquid or fluid, i.e., the medicament, is provided at an upper section of the nebulizer housing 3. To get filled by said liquid or fluid, the reservoir 9 of the inhalation therapy device 1 comprises an opening 21. More specifically, the reservoir 9 comprises a circumferential rim 27 which delimits the reservoir 9 from the environment.

A lid 10 comprising a lid body 22 is provided on the upper section of the reservoir 9, i.e., on the circumferential rim 27, for ensuring that the fluid or liquid in the reservoir 9 is not spilled during therapy. Hitherto, the circumferential rim 27 of the reservoir 9 defines the opening 21 upon placing of the lid 10 on the reservoir 9. Accordingly, the lid 10 is provided for sealing the reservoir's opening 21, for example, via threads that provoke a reversible connection of the lid body 22 and the reservoir 9 arranged in the nebulizer housing 3.

To produce the aerosol for therapy, the membrane unit 5 is disposed in the housing body 2, specifically in the nebulizer housing 3, more specifically in the flow path 8 extending through the nebulizer housing 3.

The membrane unit 5 comprises an aerosol-generating membrane 11 and an actuator 12. In Fig. 3, the membrane unit 5 is not illustrated for easier orientation.

In the cross-sectional view of Fig. 2, the aerosol-generating membrane 11 is arranged in an upright position, i.e., in a vertical position, in the nebulizer housing 3, such that the aerosol-generating membrane 11 can encounter the fluid or liquid being held in the reservoir 9. In other words, the reservoir 9 and the aerosol-generating membrane 11 are disposed adjacent to each other, such that the fluid or liquid can be supplied to a first side 11.1 of the aerosol-generating membrane 11, when a fluid or liquid is held in the reservoir 9. Doing so enables that the fluid or liquid is automatically, i.e., gravitationally, transported to the first side 11.1 of the aerosol-generating membrane 11 of the membrane unit 5 for producing aerosol for the user's therapy. This is achieved by arranging the aerosol-generating membrane 11, preferably, at a lowermost position of the reservoir 9 when the inhalation therapy device 1 is held by a user during therapy (see Fig. 2).

The aerosol-generating membrane 11 comprises a plurality of (non-illustrated) apertures and the actuator 12 is coupled to the aerosol-generating membrane 11, such that it can vibrate the same. By doing so, the liquid passes through the apertures, and aerosol is generated at a second side 11.2 of the aerosol-generating membrane 11 opposite of the first side 11.1 thereof. That is, an aerosol bolus, which may also be understood as an "aerosol plume", is generated in the chamber on the in Fig. 2 left-hand side of the membrane unit 5 inside the nebulizer housing 3.

In consequence of said aerosol being generated at the second side 11.2 of the aerosol-generating membrane 11, a negative pressure builds up in the reservoir 9, which is closed by the lid 10. The more aerosol gets generated, the more negative the pressure in the reservoir 9 gets.

Said negative pressure is desired in order to ensure that the entire fluid gets aerosolized by the membrane unit 5. Yet, if said amount of underpressure builds up to high, i.e., it gets too strong (= too negative), it may also be disadvantageous for the aerosol generation, especially because this may result in liquid droplets, which cannot be used as an aerosol for therapy. As such, it is desired to keep the amount of negative pressure in the reservoir 9 in a certain range.

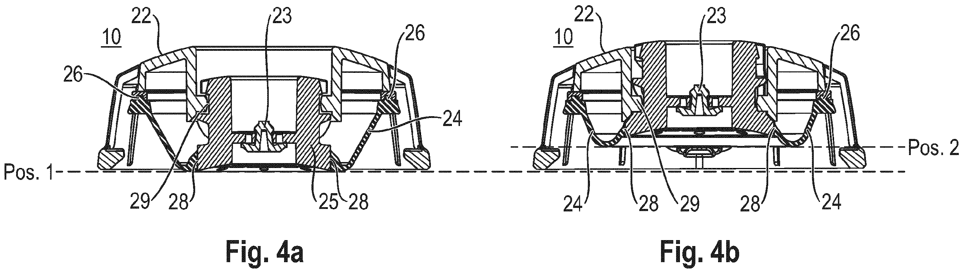

To do so, the present embodiment is provided with a valve, here a check valve 23, which is arranged in the lid 10. Said check valve 23, which is formed as an umbrella valve in the exemplary embodiment (see, e.g., Figures 2, 4a and 4b), is configured to open when the amount of negative pressure in the reservoir 9 reaches a threshold upon vibration of the membrane and is configured to close when the amount of negative pressure drops below the threshold, i.e., when the negative pressure is reduced, meaning it becomes more positive (less negative).

Extensive experimental studies have shown that the optimum therapy results and the highest TOR can be obtained by using a valve opening and closing threshold of -100mbar or -200mbar depending on the liquid or fluid to be aerosolized. In the present embodiment, the threshold which triggers the opening and, accordingly, also the closing of the check valve 23, is set to -100mbar. As such, the negative pressure in the reservoir 9, which builds up upon aerosol generation by the vibration of the aerosol-generating membrane 11 via the actuator 12 can be prevented from being to extensive, i.e., too negative. At the same time, it can be achieved that a certain amount of underpressure, which is below the threshold amount, can be maintained during therapy in order to avoid any "bleeding" effects at the second side 11.2 of the aerosol-generating membrane 11.

As mentioned earlier, the check valve 23 of the exemplary embodiment is constituted as an umbrella valve.

As the name already suggests, the umbrella valve, just like an (at least partially) opened umbrella protecting the user from rain or sun, is an elastomeric valve component, which comprises a central stem and a radially extending, convex diaphragm shaped valve disk, which may also be understood as a sealing disk, and which acts against a valve seat as the check valve's counterpart upon closing the valve 23 and holding the same in a closed state. It is, in this connection, apparent from Figures 2, 4a and 4b that the lid body 22 acts as the valve seat in this configuration.

This elastomeric valve component, i.e., the umbrella valve, is used as a sealing element acting as a backflow prevention device or one-way/check valve and can, thus, be understood as a vent valve, pressure relief valve, or metering valve that helps to maintain the desired amount of negative pressure inside the reservoir 9 during therapy.

When acting against the valve seat, the convex diaphragm of the umbrella valve flattens out against the valve seat and absorbs a certain amount of seat irregularities and reliably creates a sealing force. Thus, the umbrella valve enables a forward flow once the head pressure creates enough force to lift the convex diaphragm from the valve seat and so it will allow flow at a predetermined pressure in one way and prevent back flow immediately in the opposite way.

The main advantage compared to other types of valves, such as spring-loaded disc valves, is that the umbrella valve uses its elastic material properties and its preloaded convex shape to create the sealing force against the valve seat and that it uses the central stem to hold the component in place so as to avoid the need for additional components such as a spring and the need for a central or circumferential disc positioner(s). This simplifies the design of the assembly and makes the valve adaptable to minimal space, reduces the number of pieces in the valve, simplifies its assembly, and last but not least is very cost effective.

That is, the configuration of the check valve 23 as an umbrella valve enables to obtain a simple, consistent, and reliable positioning and, thus, a reliable opening and closing of the valve 23 on the valve seat around the predetermined threshold with a simple configuration. This interaction of the valve seat and the umbrella valve accordingly results in a reliable and consistent maintenance of a desired amount of negative pressure inside the reservoir 9 and, thus, an improved inhalation therapy.

In the exemplary embodiment illustrated, among others, in Figures 2, 4a and 4b, the check valve 23 is the umbrella valve UM 070.006-141.01 or UM 070.006-171B.01 of MiniValve International having a valve disc diameter of 7 mm and a total (stem) height of 4.5mm. Yet, it is also possible to use other umbrella valves, or even different (check) valve types depending on, e.g., the environmental conditions given, the fluid or liquid to be used, and the inhalation therapy device configuration chosen.

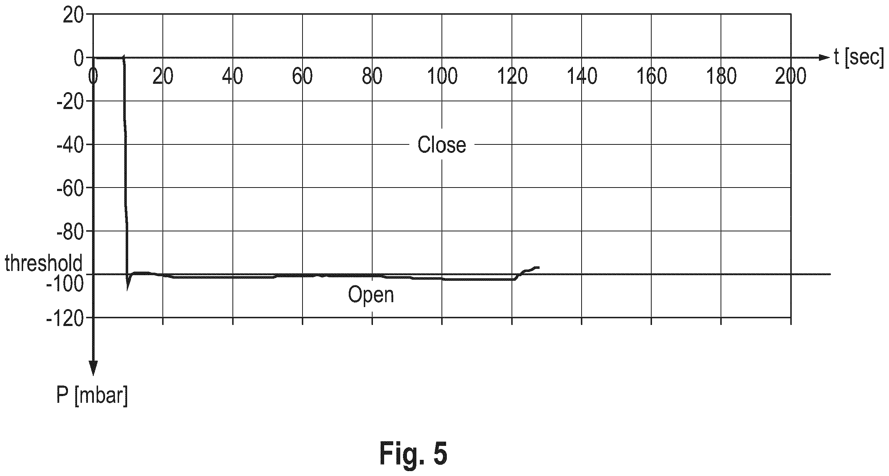

In line with the aforesaid, Figure 5 provides a diagram, which exemplarily illustrates the negative pressure curve inside the reservoir 9 in the inhalation therapy device 1 over a single administration interval.

In detail, Figure 5 illustrates the pressure p, specifically the negative pressure (underpressure) prevailing inside the reservoir 9 closed by lid 10 in mbar on the y-axis and the therapy time t in seconds on the x-axis.

It can be taken from said Figure 5 that the pressure inside the reservoir 9 is the environmental pressure, i.e., 0, for the first 10 seconds. This reflects a situation, in which the lid 10 does not yet close the reservoir 9, for example, during filling liquid or fluid into said reservoir 9.

Once the liquid or fluid is poured into the reservoir 9, the lid 10 is closed, and the aerosol generation by the vibration of the aerosol-generating membrane 11 via the actuator 12 is started at the beginning of the therapy after said first 10 seconds, the amount of negative pressure inside the reservoir 9 begins to build up and underpressure is generated in the reservoir. As mentioned earlier, the valve 23 in the lid 10 is configured to open when the threshold, here -100mbar, is met.

As illustrated in Figure 5, the negative pressure exceeds the underpressure threshold very shortly up to approximately 108 mbar due to the inertia of the valve actuation. The pressure graph of Figure 5 then continues in slight waves (overshoot and undershoot) and ideally evens out during the further course of the administration interval due to the operation, i.e., the opening and closing, of the valve and its configuration as described above for 110 seconds of therapy (120 seconds in total). Thus, it is possible to maintain the amount of negative pressure inside the reservoir 9 at/around a certain underpressure level, here -100mbar.

Figure 5 also shows a slight drop of the pressure inside the reservoir 9 after 120 seconds from approximately 102mbar to 96mbar. This is due to the fact that after 120 seconds, i.e., 110 seconds of aerosol generation, almost the entirety of exemplarily used amount of fluid or liquid, here NaCl, is aerosolized, such that the apertures of the membrane are not entirely covered by liquid on the first side of the membrane 11.1 anymore and air can pass through said apertures.

To prevent that the above-described positive effects on the aerosol generation caused by the controlled negative pressure range inside the reservoir 9 due to the configuration of the check valve 23 in the lid 10 can only be obtained after the negative pressure has been build up to a sufficient level after the aerosol generation has been started, the inhalation therapy device 1 further comprises a negative pressure generating device.

Said negative pressure generating device is configured to generate an initial negative pressure in the reservoir 9 before the aerosol-generating membrane 11 is vibrated and, accordingly, aerosol gets generated. In other words, said negative pressure generating device allows to start the generation of aerosol with a negative pressure being present in the reservoir 9. Accordingly, the improved aerosol generation can be achieved from the very first moment of vibrating the aerosol-generating membrane 11 without the above-mentioned risk of "bleeding" aerosol that passes through the apertures of the aerosol-generating membrane 11 without being aerosolized.

For generating said initial negative pressure in the reservoir 9 before the aerosol-generating membrane 11 is vibrated, it is, among others, apparent from Figures 2, 4a and 4b that the negative pressure generating device comprises an annular sealing 24 and a moving member 25. In this connection, Figures 4a and 4b illustrate the lid 10 of the inhalation therapy device with its valve 23 and the initial negative pressure generating device arranged therein. The circumferential rim 27 of the reservoir 9 as well as the remainder of nebulizer housing 3 is not illustrated in Figures 4a and 4b for easier orientation.

The annular sealing 24 comprises an outer circumferential portion 26 and an inner circumferential portion 28.

The outer circumferential portion 26 of the annular sealing 24 is sandwiched between the lid body 22 of the lid 10 and the circumferential rim 27 of the reservoir 9 and, accordingly, delimits and seals the lid 10 to the environment in the region of the opening 21. Put differently, the outer circumferential portion 26 of the annular sealing 24 seals the contact region of the circumferential rim 27 of the reservoir 9 and the lid body 22 during therapy. As such, any leakage of fluid or liquid in the contact region between the lid body 22 and the reservoir 9 can be reliably prevented.

The opposing inner circumferential portion 28 of the of the annular sealing 24 is attached to the moving member 25.

Accordingly, the annular sealing 24 interconnects and seals the region between the lid body 22 and the moving member 25 in a flexible and movable manner. Put differently, even when the position of the moving member 25 is changed to generate the initial negative pressure, which will be described in more detail below, the reservoir 9 can be reliably sealed to the environment via the lid 10 and the annular sealing 24.

In the illustrated embodiment, the moving member 25 forms the centre portion of the negative pressure generating device. Yet, the present embodiment is not limited to such a centred arrangement of the negative pressure generating device in the lid 10.

To generate the initial negative pressure via said negative pressure generating device, the moving member 25 is configured to move the inner circumferential portion 28 of the annular sealing 24 away from the aerosol-generating membrane 11, specially away from the first side 11.1 of the aerosol-generating membrane 11 upon fastening of the lid body 22 to the reservoir 9. That is, said configuration of the moving member 25 and the caused movement away from the aerosol-generating membrane 11 allows to increase the sealed volume for the liquid or fluid and the air interlocked and sealed in the reservoir 9 via the interconnected lid 10 and by said movement provokes the negative pressure even when the actuator 12 is not operated. In Fig. 2, an exemplary movement direction D of the inner circumferential portion 28 of the annular sealing 24 upon fastening of the lid body 22 to the reservoir 9 to generate the initial negative pressure is illustrated by arrow D. It is apparent that the moving member 25 moves said inner circumferential portion 28 of the annular sealing 24 away from the nebulizer housing 3 towards the upper side of the inhalation therapy device 1.

The operating mechanism of the negative pressure generating device that allows to generate an initial negative pressure inside the reservoir 9 before the membrane unit 5 begins to operate is already known from Figures 3A and 3B and the corresponding description passages of the Applicant's previous application EP 4 249 022 A1, which is incorporated herein by reference.

For the sake of completeness, the operation of the negative pressure generating device is briefly explained with a reference to the cross-sectional views of Figures 4a and 4b of the present disclosure.

The movable member 25 is movably linked to the lid body 22 via the annular sealing 24. As illustrated in Figures 4a and 4b, the movable member 25 is connected to the inner circumferential portion 28 of the annular sealing 24 and the outer circumferential portion 26 of the annular sealing 24 is tightly squeezed between the circumferential rim 27 of the reservoir 9 and the lid body 22. In other words, the annular sealing 24 is sandwiched/clamped between the circumferential rim 27 of the reservoir 9 and the lid body 22 due to the threaded connection of the circumferential rim 27 and the lid body 22. Thus, the opening 21 can be sealed to the environment.

With regard to the negative pressure generation via the negative pressure generating device, Figure 4a illustrates the movable member 25 in a position 1 and Figure 4b illustrates the movable member 25 in a position 2. It is apparent from a comparison of these two positions, that the movable member 25 moves with the annular sealing 24 from the position 1 in Figure 4a to the position 2 in figure 4a upon attachment of the lid body 22 to the nebulizer housing 3, wherein a negative pressure may be created in the reservoir 9. This is achieved because the movable member 25 and the lid body 22 are also threadedly engaged via an underpressure generating thread 29. As the movable member 25 cannot rotate together with the lid 10 once the annular sealing 24 is sandwiched between the circumferential rim 27 and the lid body 22, the rotating of the lid 10 is transferred into a translational movement from the position 1 in figure 4a to the position 2 in figure 4b and the (initial) negative pressure is, hence, created even without operating the membrane unit.

The check valve 23 is arranged in the moving member 25 and accordingly moves together with the moving member 25 and the inner circumferential portion 28 of the annular sealing 24 upon fastening of the lid body 22 to the reservoir 9.

It is furthermore apparent, among others, from Figure 2, that, to entrain and deliver the generated aerosol at the second side 11.2 of the aerosol-generating membrane 11 inside the nebulizer housing 3 of the inhalation therapy device 1 to the user's respiratory tract, the inhalation therapy device 1 comprises the flow path 8.

The flow path 8 comprises the first opening 7 to the outside of the housing body 2, particularly the nebulizer housing 3 at one end thereof and a second opening 13 to the outside of the housing body 2, particularly the nebulizer housing 3 at another end of said nebulizer housing 3.

In the illustrated embodiment of Fig. 2, the first opening 7 of the flow path 8 and the second opening 13 are arranged at opposing sides of the nebulizer housing 3. Yet, said arrangement is not binding and it may also be possible that, for example, the position of the second opening 13 is arranged offset from a longitudinal axis through the first opening 7 of the flow path 8.

As mentioned earlier, it is the first opening 7 that is to be brought into contact, may it be directly or indirectly, for example, via a mouthpiece or a mask, with the user's respiratory tract via his or her mouth, and optionally his or her nose, to enable inhalation and exhalation through the flow path 8 of the inhalation therapy device 1 during the aerosol therapy.

Vice versa, the second opening 13 allows air to enter the flow path 8 and to be expelled therefrom. Hence, the flow path 8 and its first opening 7 and second opening 13 to the outside of the nebulizer housing 3 enable that a flow is generatable in the first flow direction A from the second opening 13 to the first opening 7 in the flow path 8 upon inhalation of the user at the first opening 7. This flow may be understood as an inhalation flow, wherein the entire flow volume along the first flow direction A to the first opening 7 passes through the second opening 13 into the flow path 8 past a measurement passage 15, which will be described in more detail below, and the membrane unit 5, and merges with the aerosol at the second side 11.2 of the aerosol-generating membrane 11 before it gets expelled from the inhalation therapy device 1 into the user's body at the first opening 7 of the flow path 8.

In the embodiment illustrated, for example, in Fig. 2, also the entire flow volume along the second flow direction B, i.e., the direction of the exhalation flow from the first opening 7 to the second opening 13, passes through the second opening 13 upon exhalation of the user at the first opening 7 into the flow path 8.

Fig. 2 illustrates the first flow direction A from the second opening 13 to the first opening 7, i.e., the direction of the inhalation flow, and the second flow direction B from the first opening 7 to the second opening 13 in the flow path 8, i.e., the direction of the exhalation flow.

Regarding the shape of the flow path 8, it is apparent from Fig. 2 that the flow path 8 is shaped to prohibit a direct (unredirected) streaming of air from the second opening 13 to the first opening 7 of the flow path 8 and vice versa. Such an exemplary flow through the flow path 8 in the nebulizer housing 3 is illustrated by line C in Fig. 2. It is apparent therefrom that a centre point of the cross-section of the first opening 7, a centre point of the cross-section of the membrane unit 5, and a centre point of the cross-section of the second opening 13 do not align with each other in one single axis, thereby provoking a redirection of the air through the inhalation therapy device 1, and thereby omitting a pipe-shaped flow path structure.

The membrane unit 5 is disposed in the flow path 8, and thereby in said flow generatable by the user's breathing, especially his or her inhalation and exhalation, in such a manner that it can be surrounded by the flow through the flow path 8. As illustrated, the membrane unit 5 is arranged in an axially and circumferentially centred position of the flow path 8, such that a sheathing enveloping flow can pass the entire of the membrane unit 5 inside the flow path 8 during inhalation and exhalation of the user by breathing through the inhalation therapy device 1 during therapy.

The line C extending from the second opening 13 to the first opening 7 represents an exemplary flow of the air along the first flow direction A (i.e., an inhalation (air) flow). It should, however, be understood that this exemplary flow path 8 is not limited to the flow passing underneath the membrane unit 5 passed a lowermost position of the flow path 8. This is merely one example of a flow through the cross-sectional illustration of the flow path 8. In reality, the flow passes the membrane unit 5 and the illustrated reservoir 9, for example, also on the sides of the membrane unit 5, as it is arranged in such a manner that an envelope flow is generated about the circumference of the membrane unit 5.

It is the air flow that is redirected to be guided around the membrane unit 5 for entraining and delivering the generated aerosol upon inhalation.

To trigger the actuator 12 of the membrane unit 5 and to evaluate the quality of the inhalation therapy, it is required to observe the way the inhalation and the exhalation through the inhalation therapy device 1 is performed. Hitherto, the preferred embodiment provides an easy to manufacture and disinfect solution that reliably monitors/measures the pressure and negative pressure inside the flow path 8 upon inhalation and exhalation as a flow parameter inside the flow path 8.

To do so, the inhalation therapy device 1 comprises a sensor 14, which is configured to detect a pressure value inside the flow path 8.

It is apparent from the exploded isometric illustration of the inhalation therapy device 1 in Fig. 3 that the controller housing 4 accommodates said sensor 14 besides a controller, which is configured to control the membrane unit 5, i.e., which is configured to trigger the actuator 12 for generating the aerosol for therapy. Accordingly, the sensor 14 arranged in the controller housing 4 is positioned outside the flow path 8 extending through the nebulizer housing 3.

To provide the observation of the pressure values in the flow path 8 via the sensor 14, the inhalation therapy device 1 comprises a measurement passage 15, which connects the flow path 8 and the sensor 14. Given the fact that the flow path 8 extends through the nebulizer housing 3 and the sensor 14 is securely housed in the controller housing 4, the measurement passage 15 comprises a part 15.1 being formed in the nebulizer housing 3 and a part 15.2 being formed in the controller housing 4. In the preferred embodiment, the entire measurement passage 15 comprises a circular shape and the central axes of the part 15.1 being formed in the nebulizer housing 3 and the part 15.2 being formed in the controller housing 4 align with each other, such that the part 15.1 being formed in the nebulizer housing 3 and the part 15.2 being formed in the controller housing 4 form one single cylindrical channel, a hose, a pipe, or the like, which interconnects the flow path 8 and the sensor 14 without disturbing the flow mitigation in the measurement passage 15 by turbulences or the like. Accordingly, the pressures created by the inhalation and exhalation of the patient can mitigate through the flow path 8 and through the measurement passage 15 in order to be detected by the sensor 14.

The connection of the measurement passage 15, particularly the part 15.1 of the measurement passage 15 in the nebulizer housing 3 and the flow path 8 is disposed upstream of the membrane unit 5 in the flow path 8, when seen in the direction of the inhalation flow A. To illustrate the same, the membrane unit 5 is hidden in the open state of the nebulizer housing 3 illustrated in Fig. 3.

As illustrated in Figs. 2 and 3, the provision of the measurement passage 15 allows a distant arrangement of the sensor 14 from the flow path 8. The pressure sensor 14 of the exemplary embodiment is a differential pressure sensor that determines a static pressure in the flow path 8 and compares the same to the environmental pressure, i.e., a reference pressure, which may also be understood as an ambient pressure. To detect said environmental pressure, the controller housing 4 may additionally comprise a hose 16 extending through the controller housing 4 that enables a detection of said environmental pressure and a comparison of the same with the pressure in the flow path 8 via the differential pressure sensor 14.

In the exemplary embodiment, not only the measurement passage 15, but also the second opening 13 of the flow path 8 is arranged upstream of the membrane unit 5, when seen in the first flow direction A. It is, in this connection, apparent from Fig. 2 that the second opening 13 is arranged at an upper half of the nebulizer housing 3.

It is furthermore apparent from the cross-sectional view of Fig. 2 that the measurement passage 15 is disposed substantially in the middle between the membrane unit 5 and the second opening 13 in the flow path 8, when seen along the first flow direction A. However, this arrangement is not particularly binding, and it may also be possible to arrange the interconnection of the measurement passage 15 and the flow path 8 closer to the second opening 13 than to the membrane unit 5 in the flow path 8 or even to arrange the measurement passage 15 closer to the membrane unit 5 than to the second opening 13 in the flow path 8.

To protect the sensor 14 from liquids, aerosol (droplets), sputum, or the like in the best possible way and to allow a secure and reliable cleaning of the inhalation therapy device 1 even when the nebulizer housing 3 and the controller housing 4 are detached from each other, the inhalation therapy device 1 comprises two pressure-transmitting membranes 17, 18. One pressure-transmitting membrane 17 closes/seals the part 15.1 of the measurement passage 15 in the nebulizer housing 3, while the other pressure-transmitting membrane 18 closes/seals the part 15.2 of the measurement passage 15 in the controller housing 4.

To keep any pressures losses when transmitting the pressure mitigating through the flow path 8 and through the measurement passage 15 towards the sensor 14 as low was possible, the pressure-transmitting membranes 17, 18 are respectively configured to transmit the pressure in the flow path 8 to the sensor 14, wherein the pressure-transmitting membrane 17 arranged in the nebulizer housing 3 is impermeable to liquid, while the pressure-transmitting membrane 18 arranged in the controller housing 4 is impermeable to liquid and air.

It is apparent from Fig. 3 that the sensor 14 in the controller housing 4 is not in direct contact with the pressure-transmitting membrane 18, which closes the part 15.2 of the measurement passage 15 in the controller housing 4 between the sensor 14 and said pressure-transmitting membrane 18. To the contrary, the pressure-transmitting membrane 18 and the sensor 14 are distant to each other. The volume of said part of measurement passage 15 created by said distance is filled by a gel in order to eliminate potential pressure losses by a compressible medium, such as air, which would fill the volume when the gel would not be present.

The second opening 13 may be shaped in such a manner that it can be considered as a flow restrictor. To achieve the flow resistance, a geometrical form is to be established that provokes a higher flow resistance in the inhalation flow direction than in the exhalation flow direction. Typical relationships of the inhalation flow resistance to the exhalation flow resistance established by the second opening's shape are 125%, 130%, 150%, 175%, 190% or even 300%, 400%, or 500%. In a most preferred embodiment, the relationship of the inhalation flow resistance to the exhalation flow resistance established by the second opening's shape is 190%. This enables to advantageously prolong the inhalation time, while the exhalation time can be shortened.

The actual shape of the second opening 13, which is responsible to consider it as the above-mentioned flow restrictor, will now be described in more detail with reference to Fig. 2.

To do so, the flow path's second opening 13 is shaped as a fixed geometry passive valve. The second opening 13 may be formed as an integral part of the nebulizer housing 3 or as a separate structural element being connected to the nebulizer housing 3 and forming the outlet of the flow path 8 through said nebulizer housing 3 for delivering the generated aerosol to the user during therapy.

It can be taken from Fig. 2 that the cross-sectional shape of the second opening 13 continuously decreases along the second flow direction B. This continuously decreasing cross-sectional shape of the second opening 13 along the second flow direction B enables that the flow resistance in the second flow direction B can be made lower than in the first flow direction A without the need of any active elements, such as actuated valves or the like.

In another embodiment, it may also be possible that another, third opening 19 to the outside of the nebulizer housing 3 is provided in the flow path 8 between said first opening 7 and the second opening 13.

This is, for example, illustrated in Fig. 1. The third opening 19 is arranged upstream of the membrane unit 5 when seen in the first flow direction A. Further, the third opening 19 is provided with a check valve 20 of the third opening 19, which is configured to restrict a flow through the third opening 19 in the first flow direction A. That is, the check valve 20 of the third opening 19 prohibits any flow through the third opening 19 of the flow path 8 upon inhalation. However, the check valve 20 of the third opening 19 is also configured to enable a flow through the third opening 19 in the second flow direction B from the first opening 7 to the second opening 13 for enabling at least a partial, preferably substantially a full expel of air through the third opening 19.

This configuration allows to achieve the above-desired inhalation flow resistance and, at the same time, allows to reduce the exhalation flow resistance even further, as the check valve 20 of the third opening 19 facilitates the expel of air via the check valve 20 of the third opening 19 and the third opening 19 of the flow path 8.

Irrespective of the presence of a third opening 19 or not, the inhalation therapy device 1 may, in a further, not illustrated embodiment, comprise an air filter device, which is reversibly attachable and detachable to the nebulizer housing 3. Said air filter device is configured to filter harmful substances from the exhalation flow. The exhalation flow may contain substances and/or aerosols, which may contain materials or germs that may be harmful for the environment. Provided that the third opening 19 is present, the air filter device is shaped to cover said third opening 19 and the check valve 20 of the third opening 19, such that the flow in the second flow direction B primarily passes through the air filter device to the outside of the nebulizer housing 3 upon exhalation. Vice versa, if the third opening 19 is not present and the flow path 8 extends between the first opening 7 and the second opening 13 only, the air filter device is shaped to cover the second opening 13, which is, besides the first opening 7, the only opening of the flow path 8 to the outside of the nebulizer housing 3, such that the entire flow volume along the first flow direction A passes through the air filter device upon inhalation, and, upon exhalation, the entire flow volume along the second flow direction B passes through the air filter device.

### Reference Signs List

- 1: inhalation therapy device
- 2: housing body
- 3: nebulizer housing
- 4: controller housing
- 5: membrane unit
- 6: latch
- 7: first opening of the flow path
- 8: flow path
- 9: reservoir
- 10: lid
- 11: aerosol-generating membrane
- 11.1: first side of the aerosol-generating membrane
- 11.2: second side of the aerosol-generating membrane
- 12: actuator
- 13: second opening of the flow path
- 14: sensor (pressure sensor)
- 15: measurement passage
- 15.1: part of the measurement passage in the nebulizer housing
- 15.2: part of the measurement passage in the controller housing
- 16: hose
- 17: pressure-transmitting membrane in the nebulizer housing
- 18: pressure-transmitting membrane in the controller housing
- 19: third opening
- 20: check valve of the third opening
- 21: opening of the reservoir
- 22: lid body
- 23: (check) valve
- 24: annular sealing
- 25: moving member
- 26: outer circumferential portion of the annular sealing
- 27: circumferential rim of the reservoir
- 28: inner circumferential portion of the annular sealing
- 29: underpressure generating thread
- A: first flow direction (inhalation flow direction)
- B: second flow direction (exhalation flow direction)
- C: exemplary flow through the flow path
- D: movement direction of the inner circumferential portion of the annular sealing caused by the moving member

## Claims

1. Inhalation therapy device (1), comprising:
a housing body (2),
a reservoir (9) for holding a liquid,
a membrane unit (5) comprising:
an aerosol-generating membrane (11) disposed in the housing body (2) and having a plurality of apertures, wherein the aerosol-generating membrane (11) is disposed so that, when liquid is held in the reservoir (9), the liquid is supplied to a first side (11.1) of the aerosol-generating membrane (11) by gravitational force, and
an actuator (12) coupled to the aerosol-generating membrane (11) for vibrating the aerosol-generating membrane (11), whereby the liquid passes through the apertures, an aerosol is generated at a second side (11.2) of the aerosol-generating membrane (11) opposite to the first side (11.1) of the aerosol-generating membrane (11), and a negative pressure builds up in the reservoir (9),
a valve (23) configured to open when the amount of negative pressure in the reservoir (9) reaches a threshold upon use of the inhalation therapy device (1), preferably upon vibration of the aerosol-generating membrane (11), and to close when the amount of negative pressure drops below the threshold.

2. Inhalation therapy device (1) according to claim 1, wherein the valve is a check valve (23).

3. Inhalation therapy device (1) according to any of the preceding claims, wherein the threshold of the amount of negative pressure is 100mbar or 200mbar.

4. Inhalation therapy device (1) according to any of the preceding claims, wherein the inhalation therapy device (1) further comprises a lid (10) for sealing an opening (21) of the reservoir (9).

5. Inhalation therapy device (1) according to claim 4, wherein the valve (23) is arranged in the lid (10).

6. Inhalation therapy device (1) according to claim 4 or 5, wherein the lid (10) further comprises a negative pressure generating device for generating an initial negative pressure in the reservoir (9) before the inhalation therapy device (1) is in use, preferably before the aerosol-generating membrane (11) is vibrated.

7. Inhalation therapy device (1) according to claim 6, wherein the lid (10) comprises a lid body (22), and
the negative pressure generating device comprises:
an annular sealing (24) sandwiched at its outer circumferential portion (26) between the lid body (22) and a circumferential rim (27) of the reservoir (9) defining the opening (21) upon placing of the lid (10) on the reservoir (9), and
a moving member (25) attached to an inner circumferential portion (28) of the annular sealing (24), the moving member (25) being configured to move the inner circumferential portion (28) of the annular sealing (24) away from the aerosol-generating membrane (11) upon fastening of the lid body (22) to the reservoir (9) to generate the initial negative pressure.

8. Inhalation therapy device (1) according to claim 7, wherein the valve (23) is arranged in the moving member (25).
